Europäisches Patentamt

European Patent Office

Office européen des brevets

(11) Veröffentlichungsnummer: **0 230 988 B1**

(12) **EUROPÄISCHE PATENTSCHRIFT**

(45) Veröffentlichungstag der Patentschrift: **15.07.92**

(21) Anmeldenummer: **87100893.4**

(22) Anmeldetag: **23.01.87**

(51) Int. Cl.5: **C07C 227/00**, C07C 229/04

(30) Priorität: **28.01.86 DE 3602378**

(43) Veröffentlichungstag der Anmeldung:
**05.08.87 Patentblatt 87/32**

(45) Bekanntmachung des Hinweises auf die
Patenterteilung:
**15.07.92 Patentblatt 92/29**

(84) Benannte Vertragsstaaten:
**BE CH DE FR GB IT LI NL**

(56) Entgegenhaltungen:
**DE-A- 952 442**
**DE-A- 2 601 461**
**US-A- 2 777 873**
**US-A- 2 822 377**

**SYNTHETIC METHODS OF ORGANIC CHEMISTRY, Band 21, 1967, Seiten 217-218, S. Karger Verlag, Basel, CH**

(73) Patentinhaber: **BASF Aktiengesellschaft Carl-Bosch-Strasse 38 W-6700 Ludwigshafen(DE)**

(72) Erfinder: **Hutmacher, Hans-Martin Rüdigerstrasse 70 W-6700 Ludwigshafen(DE)**

Erfinder: **Broecker, Franz Josef, Dr. Schwanthaler Allee 20 W-6700 Ludwigshafen(DE)**
Erfinder: **Merger, Franz, Dr. Max-Slevogt-Strasse 25 W-6710 Frankenthal(DE)**
Erfinder: **Fischer, Rolf, Dr. Bergstrasse 98 W-6900 Heidelberg(DE)**
Erfinder: **Vagt, Uwe, Dr. Paul-Neumann-Strasse 44 W-6720 Speyer(DE)**
Erfinder: **Schneider, Heinz-Walter, Dr. Brüsseler Ring 43 W-6700 Ludwigshafen(DE)**
Erfinder: **Richter, Wolfgang, Dr. Am Hüttenwingert 16 W-6706 Wachenheim(DE)**
Erfinder: **Harder, Wolfgang, Dr. Bergwaldstrasse 16 W-6940 Weinheim(DE)**
Erfinder: **Priester, Claus-Ulrich, Dr. Wiesenstrasse 18 W-6701 Meckenheim(DE)**

(54) **Verfahren zur Herstellung von 6-Aminocapronsäureestern.**

EP 0 230 988 B1

**Beschreibung**

Nach einem aus der DE-AS 10 50 343 bekannten Verfahren werden ω-Aminoalkancarbonsäurealkylester aus 5-Cyanvaleriansäurealkylester bei erhöhter Temperatur und erhöhtem Druck in Gegenwart von Ammoniak unter Mitverwendung von Nickel- oder Kobalt-Katalysatoren hydriert. Die hierbei erzielten Ergebnisse befriedigen jedoch nicht, da die Ausbeute für einen technischen Betrieb zu wünschen übrig läßt.

Nach einem anderen, aus der US-PS 2 777 873 bekannten Verfahren, erhält man 6-Aminocapronsäureester durch Umsetzen von 5-Formylvaleriansäureester mit Ammoniak und Wasserstoff in Gegenwart von Hydrierkatalysatoren sowie Alkanolen als Lösungsmitteln unter erhöhtem Druck bei Temperaturen über 100°C. Die hierbei erzielten Ausbeuten sind noch verbesserungsbedürftig, um eine technische Realisierung zu ermöglichen.

Es war deshalb die technische Aufgabe gestellt, ein Verfahren zur Herstellung von 6-Aminocapronsäureestern aus 5-Formylvaleriansäureestern zur Verfügung zu stellen, das mit hohen Ausbeuten verläuft und bei dem wenig Nebenprodukte anfallen.

Diese Aufgabe wird gelöst in einem Verfahren zur Herstellung von 6-Aminocapronsäureestern durch Umsetzung von 5-Formylvaleriansäureestern mit Ammoniak und Wasserstoff unter Mitverwendung von Alkanolen als Lösungsmitteln in Gegenwart von Hydrierkatalysatoren bei erhöhter Temperatur und unter erhöhtem Druck, wobei man die Umsetzung bei einer Temperatur von 40 bis 95°C durchführt.

Das neue Verfahren hat den Vorteil, daß es mit hohen Ausbeuten verläuft und wenig Nebenprodukte gebildet werden. Darüber hinaus bedarf das Verfahren kürzerer Reaktionszeiten. Das neue Verfahren ist insofern bemerkenswert, als es auch bei Verwendung von 5-Formylvaleriansäuremethylester und Methanol als Lösungsmittel in hohen Ausbeuten verläuft, obwohl dies ausweislich der US-PS 2 777 873, Spalte 2, Zeilen 37 bis 40, nicht zu erwarten war und hohe Ausbeuten nur durch Verwendung von sekundären oder tertiären Estern möglich sind.

Bevorzugte 5-Formylvaleriansäureester sind 5-Formylvaleriansäurealkylester, insbesondere solche von $C_1$-$C_4$-Alkanolen wie Methyl, Ethyl, Propyl, Isopropyl oder n-Butylester. Geeignete Ausgangsverbindungen sind demnach 5-Formylvaleriansäuremethylester, 5-Formylvaleriansäureethylester, 5-Formylvaleriansäurepropylester, 5-Formylvaleriansäureisopropylester oder 5-Formylvaleriansäure-n-butylester. Besondere technische Bedeutung hat 5-Formylvaleriansäuremethylester erlangt.

Die Umsetzung wird unter Mitverwendung von Alkanolen als Lösungsmittel durchgeführt. Vorteilhaft verwendet man hierbei Alkanole, die der Alkoholkomponente des 5-Formylvaleriansäureesters entsprechen. Demzufolge sind bevorzugte Lösungsmittel Methanol, Ethanol, Propanol, Isopropanol oder n-Butanol. Besonders bevorzugt ist die Kombination 5-Formylvaleriansäuremethylester/Methanol. Vorteilhaft werden die 5-Formylvaleriansäureester als 1 bis 50 gew.%ige, vorteilhaft 2 bis 35 gew.%ige, insbesondere 5 bis 25 gew.%ige, Lösung in den genannten Lösungsmitteln eingesetzt.

Im allgemeinen verwendet man je Mol 5-Formylvaleriansäureester 2 bis 50 Mol Ammoniak. Besonders gute Ergebnisse erhält man, wenn man je Mol 5-Formylvaleriansäureester 5 bis 30 Mol, insbesondere 10 bis 25 Mol, Ammoniak anwendet.

Die Umsetzung wird bei einer Temperatur von 40 bis 95°C, insbesondere 60 bis 90°C, durchgeführt.

Vorteilhaft wird je Mol 5-Formylvaleriansäureester 1 bis 20 Mol Wasserstoff angewandt. Es hat sich als vorteilhaft erwiesen, einen Wasserstoffpartialdruck von 5 bis 1000 bar, vorzugsweise 20 bis 500 bar, insbesondere 50 bis 200 bar, aufrechtzuhalten.

Bevorzugte Hydrierkatalysatoren sind Metalle der VIII. Gruppe des Periodischen Systems, insbesondere Nickel- oder Kobalt-Katalysatoren, ferner Edelmetall-Katalysatoren wie Palladium, Platin oder Rhodium. Die Katalysatormetalle können als Vollkatalysatoren, z.B. in feiner Verteilung als Raney-Nickel oder Raney-Kobalt in Suspensionsfahrweise oder magnetisch in der Reaktionszone fixiert, als Mischkatalysatoren oder auf Trägern niedergeschlagen angewandt werden. Geeignete Träger sind beispielsweise Aluminiumoxid, Kieselgel oder Magnesiumsilikate. Gut geeignet sind auch Skelett-Katalysatoren. Die katalytisch aktiven Metalle werden besonders vorteilhaft in feiner Verteilung angewandt. Deshalb haben sich Skelett-Katalysatoren als besonders geeignet erwiesen.

Besonders bevorzugt verwendet man Katalysatoren, die durch Kalzinieren von Verbindungen der Formel

$$[(Mg_a Ni(II)_b Co(II)_c)Al_2]CO_3(OH)_{16} \times 4\ H_2O\ ,$$

in der a für eine ganze oder Dezimalzahlen von 0 bis 4 steht und b und c für ganze Zahlen oder Dezimalzahlen von 0 bis 6 stehen, mit der Maßgabe, daß $2\ (a+b+c) = 12$ ist, bei Temperaturen von 200 bis 600°C und anschließende Reduktion mit Wasserstoff bei erhöhter Temperatur, z.B. von 350 bis 450°C hergestellt wurden. Besonders bewährt haben sich Katalysatoren, die erhalten wurden durch Kalzinieren und Reduzieren von

$Ni_6 Al_2 (OH)_{16} CO_3 \times 4 H_2O$
$Ni_5 MgAl_2 (OH)_{16} CO_3 \times 4 H_2O$
$CO_6 Al_2 (OH)_{16} CO_3 \times 4 H_2O$
$CO_5 MgAl_2 (OH)_{16} CO_3 \times 4 H_2O$

Die Verbindungen der Formel I erhält man beispielsweise wie folgt: Nickel, Aluminium, Kobalt und Magnesium werden in Form ihrer wasserlöslichen Salze wie Chloride, Sulfate oder vorzugsweise Nitrate, gemeinsam in Wasser gelöst und zwar in einem Mengenverhältnis, das der gewünschten Zusammensetzung des Katalysators möglichst nahe kommt und in seiner Stöchiometrie der Formel I gehorcht.

Die Metallsalzlösung soll insgesamt etwa 0,5 bis 3 molar, vorzugsweise 1,0 bis 2 molar an Metallionen sein. Sie wird auf Temperaturen von 50 bis 100°C, vorzugsweise 80 bis 100°C, erhitzt und innerhalb von 0,5 bis 10, vorzugsweise 1 bis 3 Minuten, mit einer äquivalenten Menge oder bevorzugt einem geringen Überschuß einer auf 50 bis 100°C, vorzugsweise 80 bis 100°C, erhitzten, 1 bis 3, vorzugsweise 1,5 bis 2,5 molaren Lösung eines Alkalibicarbonats vereinigt. Vorteilhaft arbeitet man mit einem Überschuß an Alkalibicarbonat der bis zu 20 Gew.%, vorzugsweise 0,5 bis 3 Gew.%, bezogen auf die theoretische Menge des Bicarbonats, beträgt. Nach der Zugabe der Metallsalzlösung wird zweckmäßig auch etwa 10 bis 30, vorzugsweise 15 bis 20 Minuten gerührt und dann der entstehende Niederschlag abfiltriert, mit Wasser gewaschen und bei Temperaturen von 50 bis 200°C, vorzugsweise 100 bis 160°C, getrocknet. Die basischen Carbonate werden in nahezu quantitativen Ausbeuten erhalten. Als Alkalihydrogencarbonate kommen insbesondere Natriumhydrogencarbonat oder Kaliumhydrogencarbonat in Betracht. Es ist aber auch möglich, Ammoniumbicarbonat bei der Fällung zu verwenden. Selbstverständlich können auch Mischungen der genannten Hydrogencarbonate verwendet werden. Außerdem ist es möglich, die Fällung der Metallionen mit Lösungen von Alkalicarbonaten wie Natriumcarbonat und/oder Kaliumcarbonat vorzunehmen, wenn man während des Fällens Kohlendioxid in die vorgelegte Alkalicarbonatlösung einleitet, was aber im Endeffekt auf eine Fällung mit Bicarbonat hinausläuft.

Vorteilhaft wendet man beim Kalzinieren Temperaturen von 250 bis 400°C für eine Dauer z.B. von 5 bis 40, insbesondere 15 bis 30 Stunden an. Vor der eigentlichen Verwendung des Katalysators wird er mit Wasserstoff vorteilhaft bei Temperaturen von 180 bis 500°C, vorzugsweise 250 bis 450°C, innerhalb von 5 bis 100 Stunden, vorteilhaft 10 bis 25 Stunden, reduziert.

Andere bevorzugte Katalysatoren sind Nickelkatalysatoren die Nickel in fein verteilter Form auf Träger, insbesondere Magnesiumsilikat, aufgebracht enthalten. Vorteilhaft haben solche Katalysatoren einen Gehalt an Nickel von 30 bis 60 Gew.%, bezogen auf die gesamte Katalysatormasse einschließlich Träger. Solche Katalysatoren werden beispielsweise erhalten nach dem in DE-Patentschrift 1 545 428 beschriebenen Verfahren.

Vorteilhaft wird Raney-Nickel oder Raney-Kobalt als Katalysator verwendet, wobei man in Suspensionsfahrweise arbeitet oder den Katalysator am Permanentmagneten magnetisch oder an Weicheisenkörpern elektromagnetisch fixiert.

Für die Umsetzung hat es sich weiterhin als vorteilhaft erwiesen, eine Verweilzeit von 1 bis 20 Minuten sowie Katalysatorbelastungen von 0,2 bis 2,0 kg 5-Formylvaleriansäureester je Liter Katalysator und Stunde einzuhalten.

Die Umsetzung kann diskontinuierlich, z.B. in einem Hochdruckgefäß, durchgeführt werden. Vorzugsweise führt man die Umsetzung kontinuierlich, z.B. in druckfesten Rührbehältern z.B. einer Rührbehälterkaskade durch. Es hat sich als vorteilhaft erwiesen, eine Rückvermischung während der Umsetzung zu vermeiden. Besonders bewährt haben sich Rohrreaktoren, in denen man die alkoholische Lösung an 5-Formylvaleriansäureester und Ammoniak über ein fest angeordnetes Katalysatorbett leitet. Besonders bewährt hat sich hierbei die Sumpffahrweise. Aus den Reaktionsausträgen wird 6-Aminocapronsäureester in üblicher Weise, z.B. durch Destillation, abgetrennt.

Das Verfahren nach der Erfindung sei an folgenden Beispielen veranschaulicht.

Beispiel 1

Ein elektrisch beheizbarer Rohrreaktor von 3 cm Durchmesser und 71 cm Füllhöhe wurde mit 500 ml eines nach DBP 2 024 282, Beispiel 3, hergestellten Nickeloxid-Aluminiumoxid-Mischkristallkatalysators, der nach dem Kalzinieren unter Zusatz von 2 Gew.% Graphit zu Pillen von 3 mm Durchmesser gepreßt wurde, gefüllt. Der Katalysator wurde anschließend in 24 Stunden bei 400°C im reinen Wasserstoffstrom reduziert.

Danach wurde unter gleichzeitigem Einleiten von Wasserstoff bei 80°C/102 bar stündlich von unten nach oben 2.200 g einer 9,86 gew.%igen methanolischen 5-Formylvaleriansäuremethylester-Lösung und 800 ml (488 g) flüssiges Ammoniak durch den Reaktor gepumpt. Vom Kopf des Reaktors gelangte das Reaktionsgemisch über einen Kühler in einen Abscheider, aus dem stündlich 2.596 g Produktgemisch (über eine Standhaltung geregelt) und 207 l Abgas ausgeschleust wurden. Der Austrag enthielt nach quantitativer gaschromatographischer Analyse 7,57 % 6-Aminocapronsäuremethylester und 0,22 % Caprolactam entsprechend Ausbeuten von 90,0 % 6-Aminocapronsäuremethylester und 3,3 % Caprolactam, bezogen auf den vollständig umgesetzten 5-Formylvaleriansäuremethylester.

Beispiel 2

Durch den in Beispiel 1 beschriebenen Reaktor wurden unter gleichzeitigem Einleiten von Wasserstoff bei 80°C/100 bar stündlich von unten nach oben 1.176 g einer 19,1 %igen methanolischen 5-Formylvaleriansäuremethylester-Lösungund 350 ml (214 g) flüssiges Ammoniak gepumpt. Über den Abscheider wurden stündlich 1.365 g Produktgemisch und 200 l Abgas ausgeschleust. Der Austrag enthielt nach quantitativer gaschromatographischer Analyse 13,98 % 6-Aminocapronsäuremethylester und 3,0 % Caprolactam, bezogen auf den vollständig umgesetzten 5-Formylvaleriansäuremethylester.

Beispiel 3

Ein vertikaler Rohrreaktor (Durchmesser 16 mm, Füllhöhe 25 cm, ölbeheizter Doppelmantel) wurde mit 50 ml eines handelsüblichen Nickelkatalysators mit 55 Gew.% Nickeloxid in feiner Verteilung auf Magnesiumsilikat gefüllt (H1-80, in Form von Strängen von 1,5 mm Durchmesser). Der Katalysator wurde in 18 Stunden unter schrittweisem Anheben der Temperatur von 60 auf 330°C und unter Steigern des Wasserstoffgehalts in dem zur Reduktion benutzten Stickstoff-Wasserstoff-Gemisch von 5 auf 50 % reduziert.

Danach wurde unter gleichzeitigem Durchleiten von Wasserstoff bei 80°C/100 bar stündlich von unten nach oben 198,5 g einer 10,0 %igen methanolischen 5-Formylvaleriansäuremethylester-Lösung und 46,4 g flüssiges Ammoniak durch den Reaktor gepumpt. Vom Kopf des Reaktors gelangte das Reaktionsgemisch über einen Kühler in einen Abscheider, aus dem stündlich 243 g Produktgemisch und 22,6 l Abgas ausgeschleust wurden. Der Austrag enthielt nach quantitativer gaschromatographischer Analyse 7,22 % 6-Aminocapronsäuremethylester und 0,15 % Caprolactam entsprechend Ausbeuten von 87,8 % 6-Aminocapronsäuremethylester und 2,4 % Caprolactam, bezogen auf den vollständig umgesetzten 5-Formylvaleriansäuremethylester.

Beispiel 4

Das Beispiel verdeutlicht den Einfluß der Temperatur auf die Hydrierausbeute.

Durch den in Beispiel 3 beschriebenen Reaktor wurden unter gleichzeitigem Einleiten von Wasserstoff bei 100 bar und verschiedenen Temperaturen stündlich von unten nach oben 98,9 g einer 10 %igen methanolischen 5-Formylvaleriansäuremethylesterlösung und 20,1 g flüssiges Ammoniak gepumpt (10 l Abgas/h). Aus den Reaktionsgemischen wurden anschließend kontinuierlich in einer Füllkörperkolonne (40 cm hoch, 2,5 cm ∅, V₂A-Maschendrahtringe von 3 mm ∅) bei 40°C durch Strippen mit Stickstoff (20 l/h) im Gegenstrom Ammoniak und ein Teil des Methanols entfernt, wonach jeweils stündlich 87,7 g Austrag anfielen.

In der nachfolgenden Tabelle sind die durch quantitative gaschromatographische Analyse (GC) bestimmten Wertproduktgehalte in den Austrägen und die daraus berechneten Ausbeuten (bezogen auf 5-Formylvaleriansäuremethylester) zusammengestellt. Die Versuche a und b sind erfindungsgemäß während der Versuch c einen nicht erfindungsgemäßen Vergleich darstellt.

## Tabelle

| Temperatur [°C] | GC-Werte [Gew.%] | | Ausbeuten [%] | |
|---|---|---|---|---|
| | 6-Aminocapronsäure-methylester | Caprolactam | 6-Aminocapron-säuremethyl-ester | Caprolactam |
| (a) 60 | 9,51 | 0,12 | 83,7 | 1,4 |
| (b) 80 | 9,78 | 0,33 | 86,1 | 3,5 |
| (c) 120 | 6,85 | 0,97 | 60,3 | 11,0 |

Beispiel 5

In einem vertikal angeordneten Rohrreaktor (Durchmesser: 14 mm, Länge: 450 mm) ist ein Stab mit einem Durchmesser von 9 mm zentral angeordnet, auf dem Permanentmagnete (mit der Feldstärke 500 Gauß) befestigt sind. Die Magnete werden mit 11,0 g Raney-Nickel beladen, indem man eine Suspension von Raney-Nickel in Wasser von unten nach oben durch den Reaktor leitet.

Danach werden unter gleichzeitigem Durchleiten von 8,7 l Wasserstoff bei 76°C und 80 bar stündlich von unten nach oben 773 g einer 12,0 gew.%igen methanolischen 5-Formylvaleriansäuremethylester-Lösung und 24 ml flüssiger Ammoniak durch den Reaktor gepumpt.

Die Ausbeute an 6-Aminocapronsäuremethylester, bezogen auf eingesetzten 5-Formylvaleriansäuremethylester, beträgt 89,1 %. Zudem erhält man 3,1 % Caprolactam bezogen auf 5-Formylvaleriansäuremethylester.

## Patentansprüche

1. Verfahren zur Herstellung von 6-Aminocapronsäureestern durch Umsetzen von 5-Formylvaleriansäureestern mit Ammoniak und Wasserstoff unter Mitverwendung von Alkanolen als Lösungsmittel in Gegenwart von Nickel-, Kobalt- oder Edelmetallkatalysatoren bei erhöhter Temperatur und unter erhöhtem Druck, dadurch gekennzeichnet, daß man die Umsetzung bei einer Temperatur von 40 bis 95°C durchführt.

2. Verfahren nach Anspruch 1, dadurch gekennzeichnet, daß man Katalysatoren verwendet, die durch Kalzinieren von Verbindungen der Formel I

$$[(Mg_aNi(II)_bCo(II)_c)Al_2]CO_3(OH)_{16} \times 4\,H_2O \,,$$

in der a für ganze oder Dezimalzahlen von 0 bis 4 steht und b und c für ganze oder Dezimalzahlen von 0 bis 6 stehen, mit der Maßgabe, daß $2 \times (a+b+c) = 12$ ist, bei Temperaturen von 200 bis 600°C und anschließende Reduktion mit Wasserstoff bei erhöhter Temperatur hergestellt worden sind.

3. Verfahren nach Anspruch 1, dadurch gekennzeichnet, daß man Nickel- Katalysatoren verwendet die 30 bis 60 Gew.% Nickel in fein verteilter Form auf Magnesiumsilikat niedergeschlagen enthalten.

4. Verfahren nach den Ansprüchen 1 bis 3, dadurch gekennzeichnet, daß man 5-Formylvaleriansäureester in Lösung mit Alkanolen und Ammoniak in Sumpffahrweise über ein fest angeordnetes Katalysatorbett leitet.

5. Verfahren nach Anspruch 1, dadurch gekennzeichnet, daß man Raney-Nickel oder Raney-Kobelt in Suspensionsfahrweise verwendet.

6. Verfahren nach den Ansprüchen 1 und 5, dadurch gekennzeichnet, daß das Raney-Nickel oder Raney-Kobalt in der Reaktionszone magnetisch oder elektromagnetisch fixiert ist.

**7.** Verfahren nach den Ansprüchen 1 bis 4, dadurch gekennzeichnet, daß man eine Verweilzeit von 1 bis 15 Minuten einhält.

**8.** Verfahren nach den Ansprüchen 1 bis 5, dadurch gekennzeichnet, daß man eine Katalysatorbelastung von 0,2 bis 2,0 kg 5-Formylvaleriansäureester je Liter Katalysator und Stunde einhält.

**9.** Verfahren nach den Ansprüchen 1 bis 6, dadurch gekennzeichnet, daß man 5-Formylvaleriansäuremethylester, gelöst in Methanol, verwendet.

**Claims**

**1.** A process for the preparation of a 6-aminocaproate by reacting a 5-formylvalerate with ammonia and hydrogen using an alkanol as a solvent and in the presence of a nickel, cobalt or noble metal catalyst at elevated temperatures and under superatmospheric pressure, wherein the reaction is carried out at from 40 to 95°C.

**2.** A process as claimed in claim 1, wherein the catalyst used is prepared by calcining a compound of the formula I

$$[(Mg_aNi(II)_bCo(II)_c)Al_2]CO_3(OH)_{16} \times 4 H_2O$$

where a is an integer or decimal number from 0 to 4 and b and c are each an integer or decimal number from 0 to 6, with the proviso that 2 (a + b + c) = 12, at from 200 to 600°C and then reducing the product with hydrogen at elevated temperatures.

**3.** A process as claimed in claim 1, wherein a nickel catalyst which contains from 30 to 60% by weight of nickel deposited in finely divided form on magnesium silicate is used.

**4.** A process as claimed in claim 1 or 2 or 3, wherein a 5-formylvalerate in solution with an alkanol and ammonia is passed over a fixed bed catalyst by the liquid phase method.

**5.** A process as claimed in claim 1, wherein Raney nickel or Raney cobalt is used in suspension.

**6.** A process as claimed in claims 1 and 5, wherein Raney nickel or Raney cobalt is fixed magnetically or electromagnetically in the reaction zone.

**7.** A process as claimed in claim 1 or 2 or 3 or 4, wherein a residence time of from 1 to 15 minutes is maintained.

**8.** A process as claimed in claim 1 or 2 or 3 or 4 or 5, wherein a space velocity of from 0.2 to 2.0 kg of 5-formylvalerate per liter of catalyst per hour is maintained.

**9.** A process as claimed in claim 1 or 2 or 3 or 4 or 5 or 6, wherein methyl 5-formylvalerate dissolved in methanol is used.

**Revendications**

**1.** Procédé de préparation d'esters de l'acide 6-aminocaproïque par la mise en réaction d'esters d'acide 5-formylvalérique avec de l'ammoniac et de l'eau, avec utilisation simultanée d'alcanols servant de solvant, en présence de catalyseurs au nickel, au cobalt ou aux métaux précieux, à température élevée et sous pression élevée, caractérisé en ce qu'on conduit la réaction à une température de 40 à 95°C.

**2.** Procédé selon la revendication 1, caractérisé en ce qu'on utilise des catalyseurs qui ont été préparés par calcination, à des températures de 200 à 600°C, de composés de formule I

$$[(Mg_aNi(II)_bCo(II)_c)Al_2]Co_3(OH)_{16} \times 4 H_2O$$

dans laquelle a est mis pour un nombre entier ou décimal de 0 à 4 et b et c sont mis pour des nombres entiers ou décimaux de 0 à 6, étant spécifié que 2 x (a + b + c) = 12, suivie de réduction avec de l'hydrogène à température élevée.

3.  Procédé selon la revendication 1, caractérisé en ce qu'on utilise des catalyseurs au nickel qui contiennent de 30 à 60% en poids de nickel déposé sous forme finement divisée sur du silicate de magnésium.

4.  Procédé selon l'une quelconque des revendications 1 à 3, caractérisé en ce qu'on fait passer l'ester d'acide 5-formylvalérique en solution avec des alcanols et de l'ammoniac, dans le mode opératoire en courant descendant, sur un lit de catalyseur disposé à l'état fixe.

5.  Procédé selon la revendication 1, caractérisé en ce qu'on utilise du nickel de Raney ou du cobalt de Raney dans le mode opératoire en suspension.

6.  Procédé selon les revendications 1 et 5, caractérisé en ce que le nickel de Raney ou le cobalt de Raney est fixé magnétiquement ou électromagnétiquement dans la zone de réaction.

7.  Procédé selon l'une quelconque des revendications 1 à 4, caractérisé en ce qu'on respecte une durée de séjour de 1 à 15 mn.

8.  Procédé selon l'une quelconque des revendications 1 à 5, caractérisé en ce qu'on observe une charge du catalyseur de 0,2 à 2,0 kg d'ester d'acide 5-formylvalérique par litre de catalyseur et par heure.

9.  Procédé selon l'une quelconque des revendications 1 à 6, caractérisé en ce qu'on utilise un ester d'acide 5-formylvalérique en solution dans du méthanol.